# EUROPEAN PATENT SPECIFICATION

(11) **EP 3 841 983 B1**
(45) Date of publication and mention of the grant of the patent: **10.01.2024**
(21) Application number: 19850967.1
(22) Date of filing: 16.07.2019
(51) Int. Cl.: A61B 8/00, A61B 8/14, A61B 8/08

(54) **ULTRASONIC ANALYSIS DEVICE, ULTRASONIC ANALYSIS METHOD, AND ULTRASONIC ANALYSIS PROGRAM**
ULTRASCHALLANALYSEVORRICHTUNG, ULTRASCHALLANALYSEVERFAHREN UND ULTRASCHALLANALYSEPROGRAMM
DISPOSITIF D'ANALYSE ULTRASONORE, PROCÉDÉ D'ANALYSE ULTRASONORE ET PROGRAMME D'ANALYSE ULTRASONORE

(30) Priority: 22.08.2018 JP 2018155356
(43) Date of publication of application: 30.06.2021
(73) Proprietor: FURUNO ELECTRIC CO., LTD., Nishinomiya-City, Hyogo 662-8580 (JP)
(72) Inventor: ARAI, Tatsuo, Nishinomiya-City, Hyogo 662-8580 (JP)
(74) Representative: Müller Hoffmann & Partner
(86) International application number: PCT/JP2019/027884
(87) International publication number: WO 2020/039796

(56) References cited:
- EP-A1- 3 323 352
- WO-A1-2017/010193
- FUKUDA OSAMU ET AL: "Analysis of thigh cross-sectional proportion using the portable ultrasound imaging system", 2016 IEEE 18TH INTERNATIONAL CONFERENCE ON E-HEALTH NETWORKING, APPLICATIONS AND SERVICES (HEALTHCOM), IEEE, 14 September 2016 (2016-09-14), pages 1-6, XP033008440, DOI: 10.1109/HEALTHCOM.2016.7749449 [retrieved on 2016-11-18]
- TANAKA NORIKO I ET AL: "Reliability of size and echo intensity of abdominal skeletal muscles using extended field-of-view ultrasound imaging", EUROPEAN JOURNAL OF APPLIED PHYSIOLOGY, SPRINGER VERLAG, HEIDELBERG, DE, vol. 117, no. 11, 14 September 2017 (2017-09-14), pages 2263-2270, XP036338520, ISSN: 1439-6319, DOI: 10.1007/S00421-017-3713-Y [retrieved on 2017-09-14]
- JUHA P AHTIAINEN ET AL: "Panoramic ultrasonography is a valid method to measure changes in skeletal muscle cross-sectional area", EUROPEAN JOURNAL OF APPLIED PHYSIOLOGY, SPRINGER, BERLIN, DE, vol. 108, no. 2, 24 September 2009 (2009-09-24), pages 273-279, XP019781273, ISSN: 1439-6327
- MACGILLIVRAY T J ET AL: "3D Freehand Ultrasound for in vivo Determination of Human Skeletal Muscle Volume", ULTRASOUND IN MEDICINE AND BIOLOGY, NEW YORK, NY, US, vol. 35, no. 6, 1 June 2009 (2009-06-01), pages 928-935, XP026756841, ISSN: 0301-5629, DOI: 10.1016/J.ULTRASMEDBIO.2008.11.013 [retrieved on 2009-01-30]
- SANTOS R ET AL: "Reproducibility of ultrasound-derived muscle thickness and echo-intensity for the entire quadriceps femoris muscle", RADIOGRAPHY, W.B. SAUNDERS, AMSTERDAM, NL, vol. 23, no. 3, 4 April 2017 (2017-04-04), XP085117165, ISSN: 1078-8174, DOI: 10.1016/J.RADI.2017.03.011
- Nishihara Ken ; Tetsuya Sakamoto: "Comparison of motor function and skeletal muscle in the elderly by analysis of musculus quadriceps femoris thickness and luminance using ultrasound images", Japanese Journal of Bone and Muscle Ultrasound, vol. 15, no. 2, 30 June 2016 (2016-06-30), pages 7 (33)-12 (41), XP009526591, ISSN: 1347-2224

## Description

### [Technical Field]

The present invention relates to an ultrasonic analysis apparatus, an ultrasonic analysis method and an ultrasonic analysis program for analyzing the inside of a body by ultrasonic waves.

### [Background of the Invention]

The decline of muscle with aging is manifested not only as a decrease in muscle mass but also as a decrease in muscle quality. There is no clear definition of muscle quality, but the main index includes increased intramuscular fat, fibrosis, fast/slow muscles, pennation angle, aerobic capacity, and neuromuscular junction functioning. Among these, intramuscular fat is the index of the muscle quality which is related to the motor function degeneracy of the elderly and needs to be measured. An increase in the intramuscular fat leads to a decrease in muscle strength that can be exerted without a decrease in muscle mass.

Conventionally, a technique for measuring the muscle quality from a muscle cross-sectional image acquired by a CT (Computed Tomography) apparatus or an MRI (Magnetic Resonance Imaging) apparatus has been used. In non-patent document 1, since there is a significant correlation between the skeletal muscle average CT value and the intramuscular fat amount obtained by muscle biopsy, it is shown that the skeletal muscle average CT value is effective as an evaluation index of the muscle quality.

### Reference Documents of Conventional Art

### [Non-Patent Document 1]

Goodpaster BH, et al, "Skeletal muscle attenuation determined by computed tomography is associated with skeletal muscle lipid content", J Appl Physiol (1985), 2000 Jul, 89 (1), 104 -10 (1), 104 -110

The paper by Fukuda Osamu et al.: "Analysis of thigh cross-sectional properties using the portable ultrasound imaging system", 2016 IEEE 18TH INTERNATIONAL CONFERENCE ON E-HEALTH NETWORKING, APPLICATIONS AND SERVICES (HEALTHCOM), 14 September 2016, pages 1 - 6, describes to compose an ultrasound image and describes that a muscle index is obtained from a cross-sectional image.

The article by Tanaka Noriko I. et al.: "Reliability of size and echo intensity of abdominal skeletal muscles using extended-field-of -view ultrasound imaging", EUROPEAN JOURNAL OF APPLIED PHYSIOLOGY, vol. 117, no.11, 14 September 2017, pages 2263-2270, is aimed to examine the reliability of extended field-of-view ultrasound imaging to evaluate the cross-sectional area and echo intensity of abdominal skeletal muscles. The publication by Juha P. Ahtiainen et al.: "Panoramic ultrasonography is a valid method to measure changes in skeletal muscle cross-sectional area", EUROPEAN JOURNAL OF APPLIED PHYSIOLOGY, vol. 108, no. 2, 24 September 2009, pages 273-279, discloses some background information regarding extended field-of-view ultrasound imaging, called panoramic ultrasonography therein.

The paper by MacGillivray T. J. et al.: "3D Freehand Ultrasound for in vivo Determination of Human Skeletal Muscle Volume", ULTRASOUND IN MEDICINE AND BIOLOGY, vol. 35, no. 6, 1 June 2009, pages 928-935, has the purpose to detect by ultrasound imaging training-induced changes in muscle cross-section area.

EP 3 323 352 A1 relates to ultrasound imaging obtaining both linear and sector images.

The publication by Santos R. et al.: "Reproducibility of ultrasound-derived muscle thickness and echo-intensity for the entire quadriceps femoris muscle", RADIOGRAPHY, vol. 23, no. 3, 4 April 2017, shows data interpolation in 3D image synthesis

### [Summary of the Invention]

However, CT and MRI systems are complex, expensive, and involve exposure to possibly harmful radiation. In addition, since there is no dedicated software for image analysis, it cannot be handled by general engineers.

An object of the present invention is to provide an ultrasonic analysis apparatus for evaluating the state of a muscle with a relatively simple configuration without being exposed to radiation.

The above objects are solved by the subject-matter of the independent claims.

An ultrasonic analysis apparatus according to the present invention as defined in independent claim 1 is characterized by comprising: an interface module configured to receive signals from a transmitter/receiver which receives ultrasonic waves transmitted from a plurality of mutually different positions on the surface of a body toward muscles inside the body and reflected from inside the body; an ultrasonic image generating module configured to generate ultrasonic images corresponding to the respective positions based on the signals inputted from the interface module; an image compositing module configured to stitch the ultrasonic images at the respective positions to generate a stitched image; an area setting module configured to both set a region of interest for at least one of the stitched image and a secondary region of interest for at least one ultrasonic image of the plurality of ultrasonic images before the stitch, the region of interest set for the stitched image and each secondary region of interest set for the at least one ultrasonic image before the stitch corresponding to each other; and an index calculation module configured to calculate an index related to the muscle, the index corresponding to a muscle quality of the muscle, from the secondary region(s) of interest set for the at least one ultrasonic image before the stitch.

An ultrasonic analysis method according to the present invention as defined in independent claim 8 is characterized by comprising the steps of receiving a signal from a transmitter/receiver which receives ultrasonic waves transmitted from a plurality of mutually different positions on a surface of a body toward muscles from inside the body and reflected therein; respectively generating ultrasonic images corresponding to the respective positions based on the received signal; stitching the ultrasonic images at the respective positions to generate a stitched image; setting both a region of interest for the stitched image and a secondary region of interest for at least one ultrasonic image of the plurality of the ultrasonic images before the stitch, the region of interest set for the stitched image and each secondary region of interest set for the ultrasonic image before the stitch corresponding to each other; and calculating an index related to the muscle, the index corresponding to a muscle quality of the muscle, from the secondary region(s) of interest set for the at least one ultrasonic image before the stitch.

An ultrasonic analysis program according to the present invention as defined in independent claim 9 is configured to cause a computer to execute processing, the computer controlling an ultrasonic analysis apparatus as defined above, which program comprises instructions, which when run on the computer, cause the ultrasonic analysis apparatus to carry out the method as defined above.

### [Effect(s) of the Invention]

According to the present invention, the state of the muscle can be evaluated with a relatively simple configuration and without being exposed to radiation.

### Brief Description of the Drawings

FIG. 1 is a block diagram of an ultrasound analysis system according to an embodiment of the present invention;
FIG. 2 illustrates an operational aspect of an ultrasound probe;
FIG. 3 is a diagram for explaining an operation mode of a linear scan mode and a sector scan mode;
FIG. 4 is a functional block diagram of a signal processing module;
FIG. 5 illustrates the generation of a linear scan image;
FIG. 6 illustrates the generation of a sector scan image;
FIG. 7 is an example of a stitched image of a thigh;
FIG. 8 is a diagram for explaining the setting of a region of interest in a stitched image;
FIG. 9 is an example of an ultrasound image in which a region of interest is set;
FIG. 10 is an example of a region of interest extracted from an ultrasound image;
FIG. 11 is a graph showing a relationship between a brightness of a region of interest and an image number;
FIG. 12 is a histogram representing a brightness distribution;
FIG. 13 is a graph showing the relationship between the brightness of the region of interest and the image number;
FIG. 14 shows a linear scan image and a two-dimensional fast Fourier transformed image of it;
FIG. 15 is a flow chart showing a flow of processing in an ultrasonic analysis method according to an embodiment of the present invention; and
FIG. 16 is a diagram for explaining another example of setting a region of interest in a stitched image.

### [Detailed Description of the Invention]

Embodiments of the present invention will now be described in detail with reference to the accompanying drawings. In the following description and the drawings, the same reference numerals indicate the same or similar components, and therefore redundant description of the same or similar components is omitted. In the following description, the quadriceps femoris is the analysis subject of muscle quality, but the type of muscle is not particularly limited in the present invention.

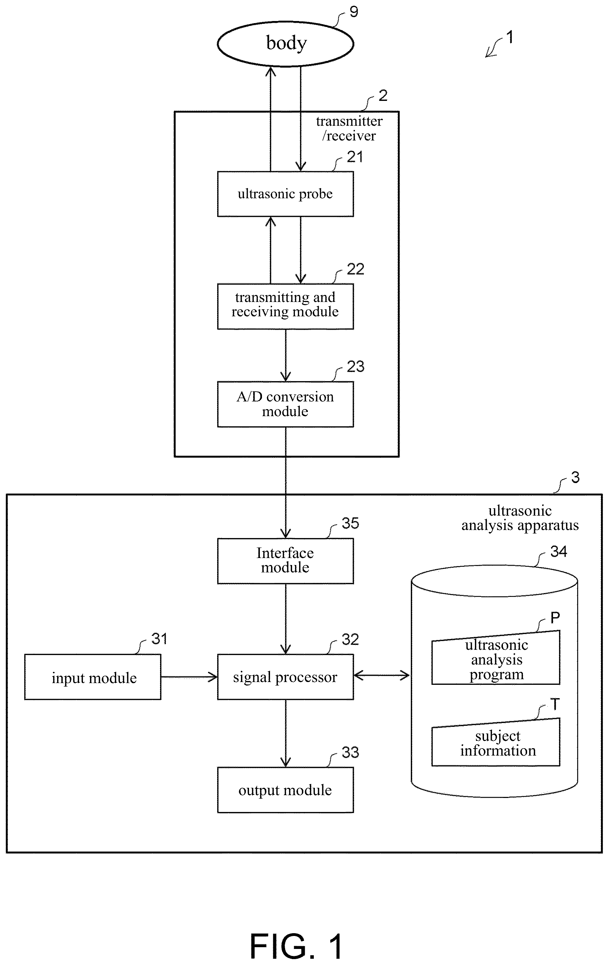

[overall configuration] FIG. 1 is a block diagram of an ultrasonic analysis system 1 according to an embodiment of the present invention. The ultrasonic analysis system 1 includes a transmitter /receiver 2 and an ultrasonic analysis apparatus 3 connected to the transmitter/receiver 2.

[transmitter/receiver] The transmitter/receiver 2 is a device for transmitting ultrasonic waves toward muscles inside a body 9 and receiving echo signals reflected from inside the body 9. The transmitter/receiver 2 includes an ultrasonic probe 21, a transmitting and receiving module 22, and an A/D conversion module 23.

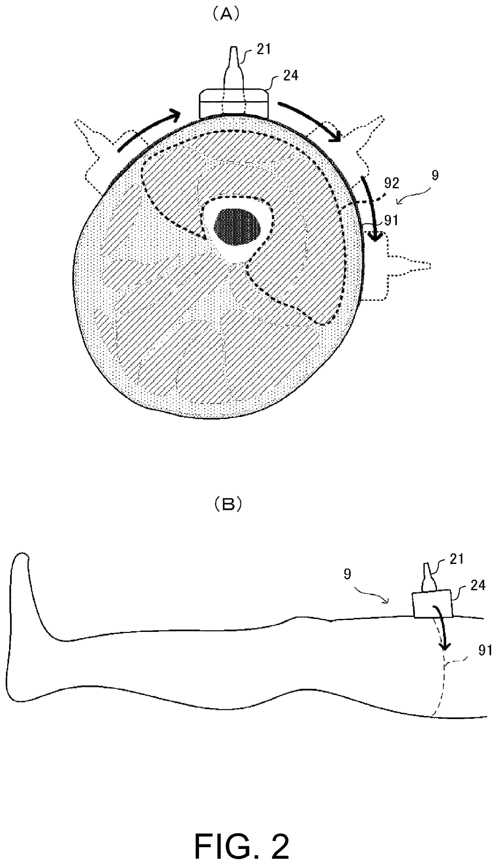

As shown in FIG. 2A, the ultrasonic probe 21 is a device for transmitting ultrasonic waves from a plurality of different positions on a surface of the body 9 toward the inside of the body 9 and receiving the ultrasonic waves reflected from the inside of the body 9. In this embodiment, the ultrasonic probe 21 is configured to be gripped and moved by a user, and an ultrasonic transmission/reception surface is provided on a lower end surface of the ultrasonic probe 21. An ultrasonic array sensor, constituted by arranging a plurality of ultrasonic vibrators in a line, is arranged on the ultrasonic transmission/reception surface. When acquiring a tomographic image of the body 9 (or a cross-sectional image), the user makes contact between the body 9 and the ultrasonic transmission/reception surface of the ultrasonic probe 21, and moves the ultrasonic probe 21 along the surface of the body 9 (scans with the ultrasonic probe 21). Meanwhile, the ultrasonic probe 21 intermittently transmits ultrasonic waves from the ultrasonic transmission/reception surface toward the inside of the body 9, and receives the ultrasonic waves reflected from the inside of the body 9 on the ultrasonic transmission/reception surface. Thus, the ultrasonic probe 21 outputs an electric signal (echo signal) indicating the received ultrasonic wave.

In an embodiment, as shown in FIGs. 2A and 2B, the ultrasonic probe 21 is attached to a probe adapter 24, and a contacting surface of the probe adapter 24 contacts on the surface of a thigh 91 of the body 9. In this state, the user moves the ultrasonic probe 21 and the probe adapter 24 along an outer periphery of a transverse section of the thigh 91 that is to be imaged. While the ultrasonic probe 21 is moved along the surface of the thigh 91, ultrasonic waves are transmitted at predetermined time intervals from the plurality of ultrasonic vibrators of the ultrasonic probe 21. Thus, the ultrasonic probe 21 transmits ultrasonic waves from different positions toward a quadriceps femoris 92 a plurality of times.

The ultrasonic probe 21 or the probe adapter 24 may be provided with an inclination sensor. The inclination sensor detects an inclination of the ultrasonic probe 21 with respect to a vertical direction, that is, a direction in which the ultrasonic transmission/reception surface is facing. Further, although an example in which the ultrasonic probe 24 to which the probe adapter 24 is attached, alternatively, the ultrasonic image may be acquired by moving only the ultrasonic probe 21 without using the probe adapter 24.

The ultrasonic probe 21 is operable in two modes, a linear scan mode for acquiring a linear scan image and a sector scan mode for acquiring a sector scan image.

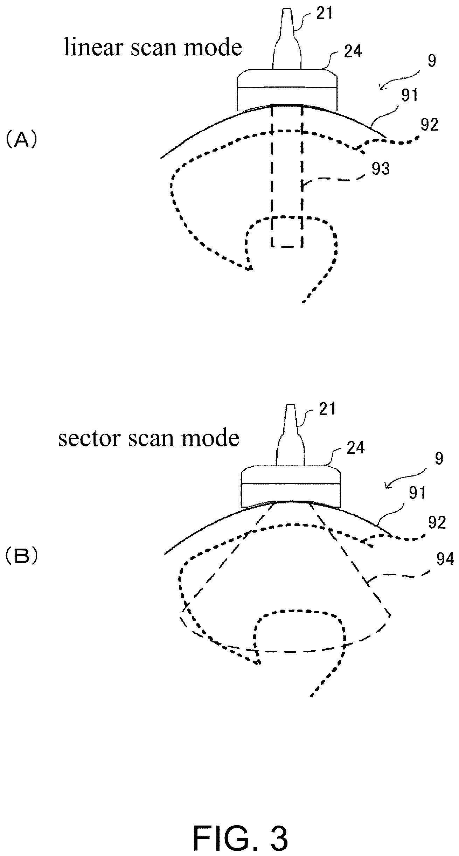

When the ultrasonic probe 21 is operated in the linear scan mode, as shown in FIG. 3A, the ultrasonic wave transmitted from the ultrasonic transmission/reception surface is directed toward a zonal region 93. Therefore, when the ultrasonic probe 21 is driven in the linear scan mode, the ultrasonic analysis apparatus 3 can generate an ultrasonic image in which the zonal region 93 is clearly imaged.

When the ultrasonic probe 21 is operated in the sector scan mode, as shown in FIG. 3B, the ultrasonic wave transmitted from the ultrasonic transmission/reception surface is directed toward a fan-shaped region 94. Therefore, when the ultrasonic probe 21 is driven in the sector scan mode, the ultrasonic analysis apparatus 3 can generate an ultrasonic image obtained by imaging the fan-shaped region 94.

In the present embodiment, the ultrasonic probe 21 operates while alternately repeating the linear scan mode and the sector scan mode, and acquires multiple linear scan images and sector scan images, for example, 200 images are acquired during the linear scan mode and the sector scan mode, thus a total of 400 images are acquired. The number of the images is only one example, and is set to an arbitrary value. The number may be determined according to the operation and the condition of the subject. The ultrasonic probe 21 may be configured to acquire either a linear scan image or a sector scan image at a given time.

The transmitter/receiver 2 shown in FIG. 1 forms a waveform of a carrier wave that constitutes a frequency of an ultrasonic band into a pulse shape to generate a transmission pulse, and outputs the generated transmission pulse to the ultrasonic probe 21. Thus, ultrasonic waves are transmitted from the plurality of ultrasonic vibrators of the ultrasonic probe 21 in a depth direction of the body 9. The transmitting and receiving module 22 receives the echo signal received by the plurality of ultrasonic vibrators of the ultrasonic probe 21 from the inside of the body 9.

The A/D conversion module 23 converts the echo signal transmitted from the transmitting and receiving module 22 from an analog format to a digital format, and transmits the converted echo signal to the ultrasonic analysis apparatus 3.

[ultrasonic analysis apparatus] The ultrasonic analysis apparatus 3 includes an input module 31, a signal processing module 32, and an output module 33.

In the present embodiment, the ultrasonic analysis apparatus 3 is constituted by a known personal computer, and includes, as a hardware configuration, a processor (not shown) such as a CPU for performing data processing, a memory (not shown) used by the processor in a work area for data processing, a recording module 34 for recording processing data, a bus (not shown) for transmitting data between each module, and an interface module (hereinafter referred to as I/F module 35) for inputting/outputting data to/from an external apparatus. As an optional function, the ultrasonic analysis apparatus 3 can be connected to an external server via a network such as the Internet.

The input module 31 receives input of an operation from a user. For example, the input module 31 may be a keyboard, a mouse, a touch panel, or the like.

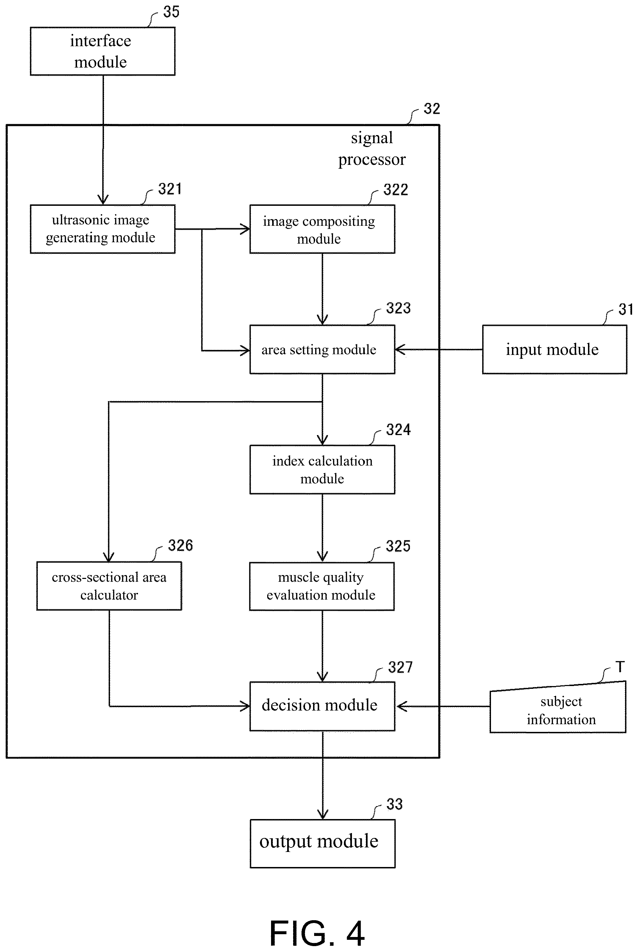

The signal processing module 32 is a functional block that receives an echo signal converted into the digital format from the transmitter/receiver 2 and performs various arithmetic processing functions such as calculation of an index corresponding to a muscle quality of the quadriceps femoris 92 to be analyzed. The signal processing module 32 may be implemented in hardware by a logic circuit formed on an integrated circuit, but in this embodiment, the signal processing module 32 is implemented in software by the processor executing an ultrasonic analysis program P previously recorded in the recording module 34 or the memory. The ultrasonic analysis program P may be installed in the ultrasonic analysis apparatus 3 via a network. Alternatively, the ultrasonic analysis program P may be installed in the ultrasonic analysis apparatus 3 by causing the ultrasonic analysis apparatus 3 to read a computer-readable non-temporary tangible recording medium, such as a CD-ROM, in which the ultrasonic analysis program P is recorded. Details of the signal processing module 32 will be described later with reference to FIG. 4.

The output module 33 outputs a calculation result of the signal processing module 32. By way of example, the output module 33 can be configured with a monitor, a printer, or the like.

The I/F module 35 receives a signal (the converted echo signal) from the transmitter/receiver 2.

FIG. 4 is a functional block diagram of the signal processing module 32. The signal processing module 32 includes an ultrasonic image generating module 321, an image compositing module 322, an area setting module 323, an index calculation module 324, a muscle quality evaluation module 325, a cross-sectional area calculator 326, and a decision module 327.

[generation of ultrasonic image and stitched image] The ultrasonic image generating module 321 generates an ultrasonic image of the inside of the body 9 based on the echo signal input from the I/F module 35. As described above, the transmitter/receiver 2 transmits ultrasonic waves a plurality of times while alternately repeating the linear scan mode and the sector scan mode, and receives the echo signals reflected from inside the body 9. Therefore, each time the transmitter/receiver 2 receives the echo signal, the I/F module 35 receives the converted echo signal. In response to this, the ultrasonic image generating module 321 generates a linear scan image as an ultrasonic image from the echo signal received in the linear scan mode, and generates a sector scan image as an ultrasonic image from the echo signal received in the sector scan mode. In the present embodiment, the ultrasonic image generating module 321 generates multiple linear scan images and multiple sector scan images, for example, 200 linear scan images and 200 sector scan images, thus, a total of 400 ultrasonic images.

The ultrasonic image generating module 321 may correct the deformation caused by the pressing of the ultrasonic probe 21 against the cross section of the body 9 in the ultrasonic image, based on a force of the ultrasonic probe 21 pressing the body 9 and a position and direction of the ultrasonic probe 21.

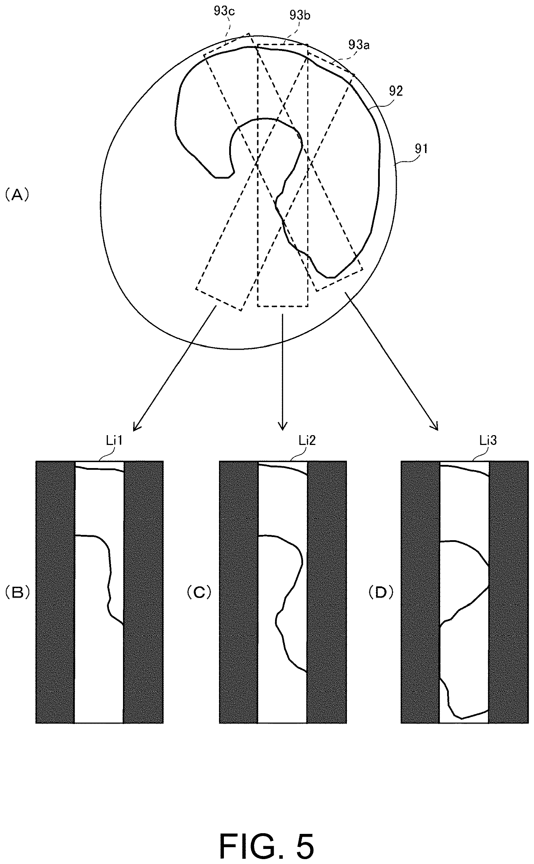

FIG. 5 is a diagram for explaining generation of a linear scan image. FIG. 5A is a partial sectional view of the thigh 91, and the thigh 91 is divided into the quadriceps femoris 92 and other portions for convenience. In the linear scan mode, since the ultrasonic wave is directed to the zonal region 93, a real image region of the linear scan image also becomes the zonal region 93. For example, when the ultrasonic wave is directed to a zonal region 93a, the linear scan image Li1 as shown in FIG. 5B is generated, when the ultrasonic wave is directed to a zonal region 93b, the linear scan image Li2 as shown in FIG. 5C is generated, and when the ultrasonic wave is directed to a zonal region 93c, the linear scan image Li3 as shown in FIG. 5D is generated.

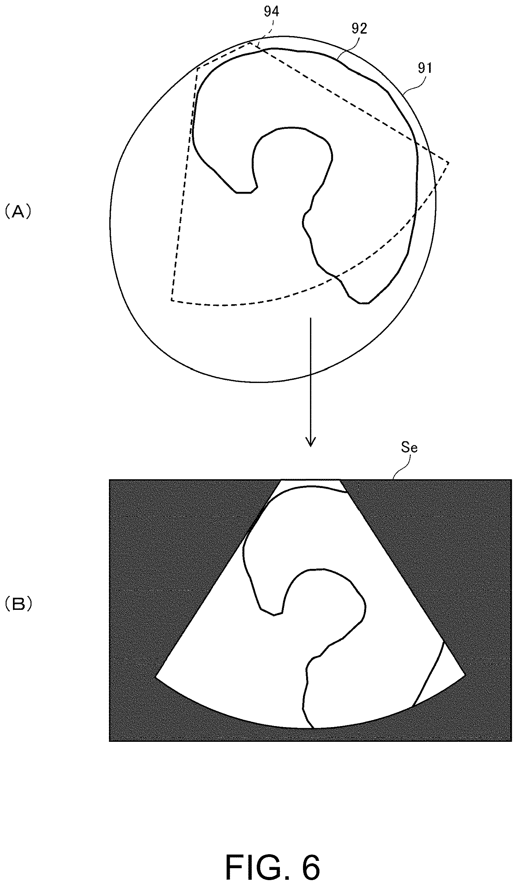

FIG. 6 is a diagram for explaining generation of a sector scan image. FIG. 6A is a partial sectional view of the thigh 91, and similarly to FIG. 5A, the thigh 91 is divided into the quadriceps femoris 92 and other portions for convenience. In the sector scan mode, since the ultrasonic wave is directed to the fan-shaped region 94, a real image region in the sector scan image Se is fan-shaped as shown in FIG. 6B.

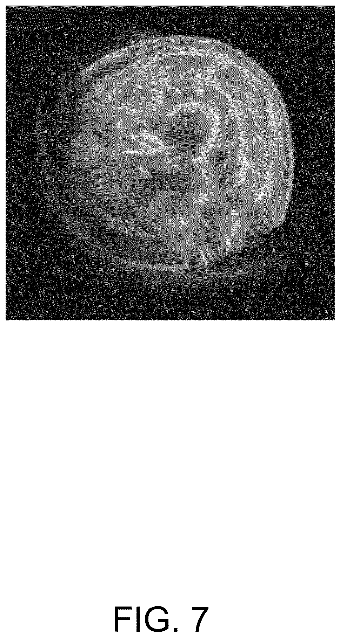

The image compositing module 322 stitches a plurality of ultrasonic images (linear scan image and sector scan image) generated by the ultrasonic image generating module 321 to generate a stitched image. The method for stitching the ultrasonic image is not particularly limited, and any known method can be used. For example, in the case of generating a stitched image by the method described in WO2017/010193A1, the image compositing module 322 detects and matches a feature amount between regions included in each of the plurality of ultrasonic images, thereby determining a position at which the plurality of ultrasonic images overlap. When the ultrasonic probe 21 or the probe adapter 24 is provided with the inclination sensor, the image compositing module 322 rotates each ultrasonic image on the basis of a detection angle obtained from the inclination sensor, and performs matching on the basis of the rotated ultrasonic image. Thus, a rotation angle of each ultrasonic image can be accurately corrected, and an overlapped position of a fragment image can be determined with higher accuracy. FIG. 7 is an example of a stitched image of the thigh 91.

[setting the region of interest] The area setting module 323 shown in FIG. 4 is a functional block for setting a region of interest (ROI: Region of Interest) for at least one of the stitched image and the ultrasonic image. In the present embodiment, the area setting module 323 is a functional block that sets a region of interest for the stitched image and sets a region of interest for the ultrasonic image corresponding to the region of interest of the stitched image before the generation of the stitched image.

The area setting module 323 may set one region of interest for the entire quadriceps femoris 92 included in the stitched image, or may evaluate the muscle qualities of each of a rectus femoris, vastus lateralis, vastus intermedius, and vastus medialis constituting the quadriceps femoris 92 in order to accurately evaluate the muscle qualities of the quadriceps femoris 92. Therefore, in this embodiment, the area setting module 323 sets a region of interest for each of the rectus femoris, vastus lateralis, vastus intermedius, and vastus medialis.

The area setting module 323 sets the region of interest in the stitched image in response to a user's operation via the input module 31. Hereinafter, a specific example of setting a region of interest in the stitched image shown in FIG. 7 will be described.

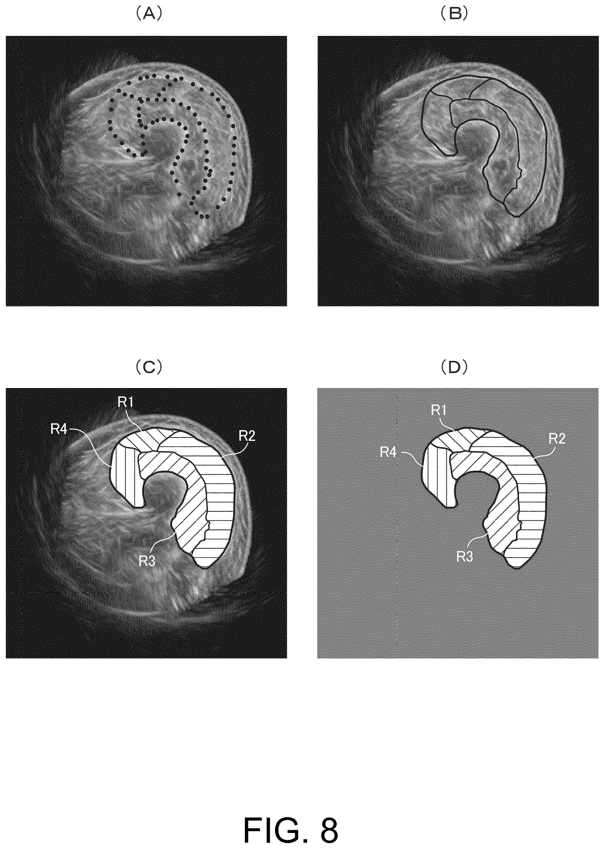

The user operates the mouse or the like while viewing the stitched image shown in FIG. 7, and as shown in FIG. 8A, hits a point at the boundary position of the rectus femoris, vastus lateralis, vastus intermedius, and vastus medialis. In this case, a color of the point may be different depending on a type of the adjacent tissue.

The points shown in FIG. 8A may be automatically set by a method for automatically detecting the boundaries of the rectus femoris, vastus lateralis, vastus intermedius, and vastus medialis.

Subsequently, as shown in FIG. 8B, in response to a user's instruction via the input module 31, points of the same color are connected to each other, whereby the signal processing module 32 creates a boundary line. Thus, as shown in FIG. 8C, the quadriceps femoris 92 is divided into 4 regions such as the rectus femoris, the vastus lateralis, the vastus intermedius, and the vastus medialis, and the area setting module 323 sets 4 regions as regions of interest R1 to R4, respectively. Thereafter, as shown in FIG. 8D, the area setting module 323 extracts a mask image (masked image) comprising the set regions of interest R1 to R4 from the stitched image. As described above, setting of the region of interest for the stitched image is completed.

The regions of interest R1 to R4 are divided by hatching, a type of hatching is independent of brightness, which will be described later.

Further, the area setting module 323 sets a region of interest corresponding to the region of interest of the stitched image also for the ultrasonic image before the stitch. In the present embodiment, the area setting module 323 overlaps the mask images composed of the regions of interest R1 to R4 extracted from the stitched image with the ultrasonic images. At this time, the mask image is rotated according to the rotation angle of each ultrasonic image. Thus, the area setting module 323 can specify the boundary of the region of interest in each ultrasonic image and also set the region of interest for each ultrasonic image.

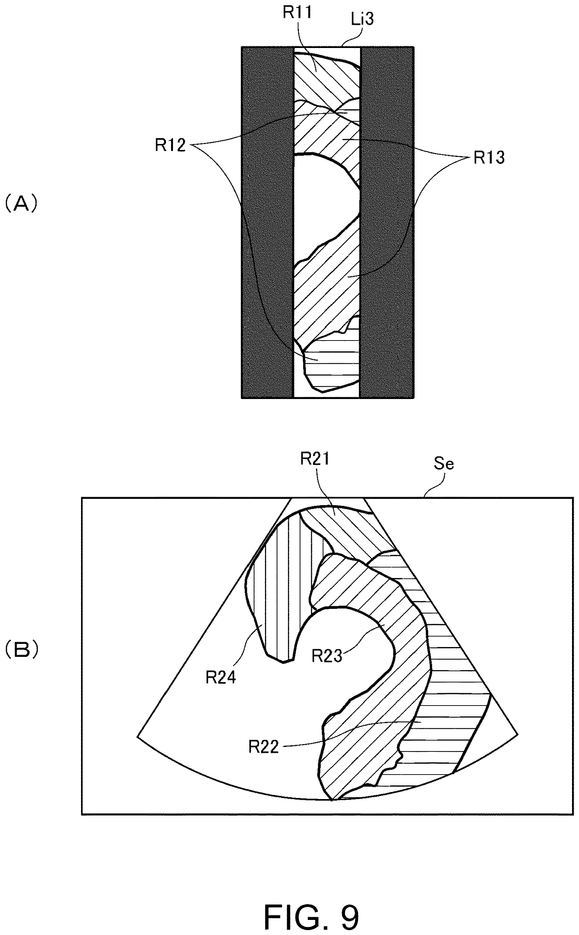

In an example, for the linear scan image Li3 shown in FIG. 5D, the regions of interest R11, R12 and R13 included in the linear scan image Li3 are set as shown in FIG. 9A. For the sector scan image Se shown in FIG. 6B, regions of interest R21, R22, R23 and R24 included in the sector scan image Se are set as shown in FIG. 9B.

In the following description, the region of interest (For example, regions of interest R11 to R13 and R21 to R24) set in the ultrasonic image before synthesis is sometimes referred to as "secondary region of interest".

[calculation of index] The index calculation module 324 shown in FIG. 4 calculates an index related to the muscle from the secondary region(s) of interest set for the ultrasonic images before the stitch. In the present embodiment, the index calculation module 324 calculates an index corresponding to the muscle quality of the muscle based on the image (here, it is an ultrasonic image, more specifically, a linear scan image or a sector scan image) corresponding to the secondary region of interest set for the ultrasonic image before synthesis. Since the brightness of the image of the muscle increases as the intramuscular fat content increases, the index calculation module 324 calculates the average value of the brightness of the secondary region of interest of the ultrasonic image as an index. A specific example of the calculation method of the index is described below.

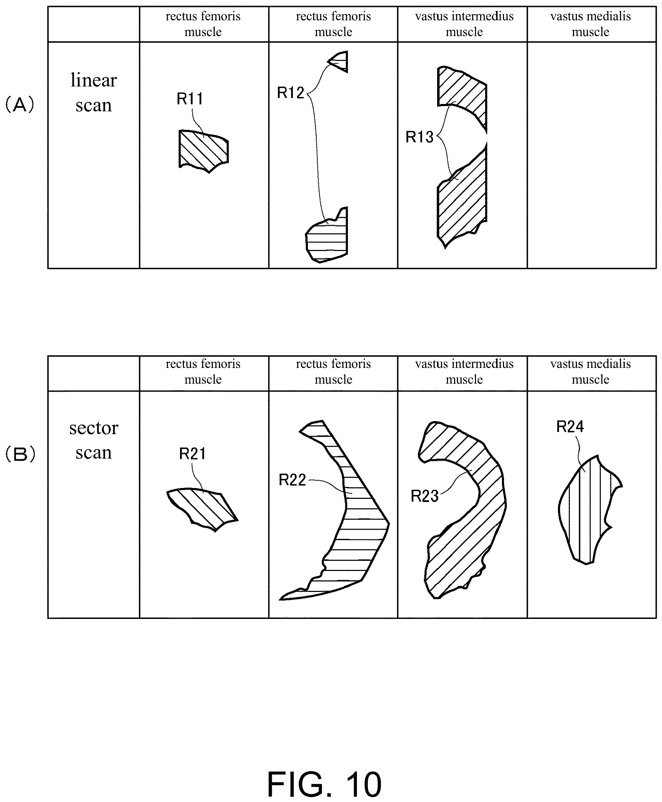

First, the index calculation module 324 extracts the secondary region of interest set in each ultrasonic image. For example, from the linear scan image Li3 shown in FIG. 5D, secondary regions of interest R11, R12, and R13 corresponding to the rectus femoris, vastus lateralis, and vastus intermedius, respectively, are extracted as shown in FIG. 10 (A). Further, from the sector scan image Se shown in FIG. 6B, secondary regions of interest R21, R22, R23, and R24 corresponding to the rectus femoris, vastus lateralis, vastus intermedius, and vastus medialis, respectively, are extracted as shown in FIG. 10 (B). The index calculation module 324 performs the extraction process for all the linear scan images and the sector scan images.

Since there is a line (Lines of fascia within bones and the vastus lateralis) with high brightness in the region of interest, an outer peripheral edge of the region of interest may be removed (For example, a few pixels) for extraction. For the removal of high brightness lines, for example, line enhancement image filters, binarization, and high brightness line detection by the Dijkstra's algorithm, can be used.

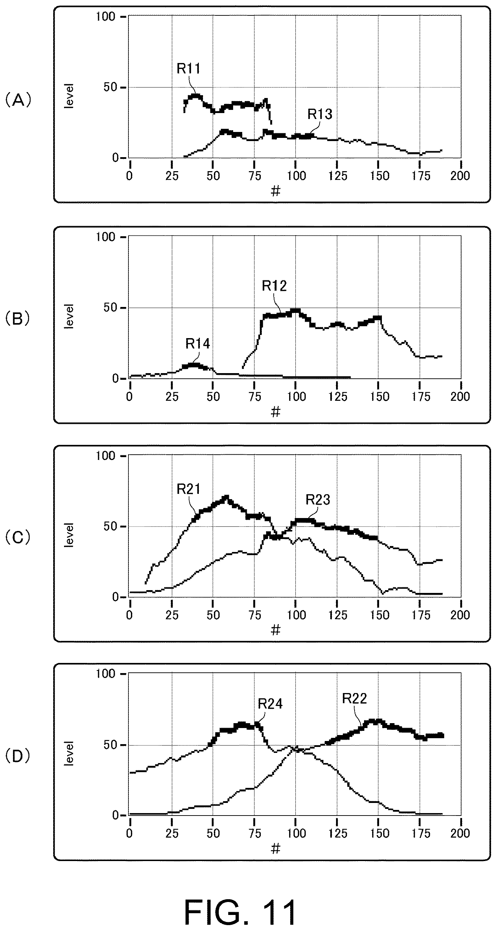

Subsequently, the index calculation module 324 calculates the brightness of each region of interest. FIGs. 11 (A) and (B) are graphs showing the brightness of the secondary regions of interest R11 to R13 extracted from the 200 linear scan images, and FIGS. 11 (C) and (D) are graphs showing the brightness of the secondary region of interests R21 to R24 extracted from the 200 sector scan images. In each graph, the horizontal axis represents a number assigned to the acquisition order of the linear scan image or the sector scan image, and the vertical axis represents the brightness of the secondary region of interest. In addition, a portion where the brightness is 70% or more of the maximum brightness of the secondary region of interest is indicated by a thick line.

The index calculation module 324 calculates an average value of brightness indicated by a thick line for each secondary region of interest, and outputs the calculated average value as an index corresponding to the muscle quality. In calculating the index, all the secondary regions of interest may be used, but the index may be calculated by selectively using either the linear scan image or the sector scan image depending on the part of the muscle.

The linear scan images and sector scan images have the following advantages and disadvantages. The linear scan image has the advantage of being less influenced by the characteristics of the echo and having high reliability. However, the linear scan image has the following disadvantages. Since the observation range at a given time is narrow and each secondary region of interest is only partially reflected, the linear scan image is easily affected by unevenness in the secondary region of interest. It is difficult to distinguish an image having a correct value from an image to be removed due to unevenness. In particular, the latter disadvantage is prominent in a linear scan image of the vastus medialis. In addition, the vastus medialis muscle has a smaller variation range than other muscles (with low sensitivity), and the effect of the directionality to which ultrasonic waves hit is greater.

On the other hand, the sector scan image has the following advantages. Since an observation range at a given time is wide and many images include all or most part of the secondary region of interest, the sector scan image is averaged even if there is unevenness in the secondary region of interest. It is easy to distinguish an image having a correct value from an image having an abnormal value (when the secondary region of interest is not reflected behind the bone). The vastus medialis muscle, which shows a large change in the linear scan image, shows the same change as other muscles in the sector scan image. On the other hand, the sector scan image is easily affected by the characteristics of the echo, and has a disadvantage that the deeper the sector scan image is, the worse the echo characteristics become.

Based on the characteristics of the linear scan image and the sector scan image as described above, an image for calculating the index may be selected for each muscle region. For the vastus lateralis and vastus intermedius in the quadriceps femoris 92, the correlation between the brightness of the secondary region of interest and the equivalent CT value tends to be higher in the linear scan image than in the sector scan image. Therefore, for the vastus lateralis and vastus intermedius, an index may be calculated using the linear scan image. On the other hand, for the rectus femoris and vastus medialis in the quadriceps femoris 92, the correlation between the brightness of the secondary region of interest and the CT value equivalent tends to be higher in the sector scan image than in the linear scan image. Therefore, for the rectus femoris and vastus medialis, an index may be calculated using the sector scan image.

The brightness can be expressed in 0 ~ 255 gradations. However, due to a possibility that an error may occur depending on the region of the muscle or the method of scanning, a brightness correction technique may be used to calculate a corrected brightness. The calculation of the corrected brightness is possible by a) correcting for scaling according to the part in order to evaluate a tendency of the part that is bright and the part that is dark appears in the same order, and b) correcting for differences of trends when using a mix of results from different scanning methods. As a result, it is not necessary to standardize settings related to image quality and brightness such as a gain of the transmitter/receiver 2.

Since the brightness is an image processing value under a specific condition, it can be generalized by converting it into a physical index. For example, brightness can be converted to dB as an echo reflection level. Specifically, a reference can be determined by a substance corresponding to 0 dB (For example, a phantom for quality control), and the brightness can be converted to dB from measurement or calculation. This makes it possible to correct variations in mass production through inspection and correction, and to standardize indices for multiple models.

Further, the index calculation module 324 may calculate a CT value equivalent value (HU), an MRI equivalent value (T1, T2), or a muscle tissue evaluation value (Fat equivalent (%), effective muscle cross-sectional area, etc.) of the image as an index corresponding to the muscle quality, instead of the average value of the brightness. These values can be correlated with clinically significant data, such as CT and MRI measurements and the actual anatomic and medical condition of the muscle.

[evaluation of muscle quality] The muscle quality evaluation module 325 shown in FIG. 4 evaluates the muscle quality based on the index calculated by the index calculation module 324. When the index is an average value of brightness, it can be evaluated that the larger the average value is, the larger the amount of fat in the muscle and lower is the muscle quality.

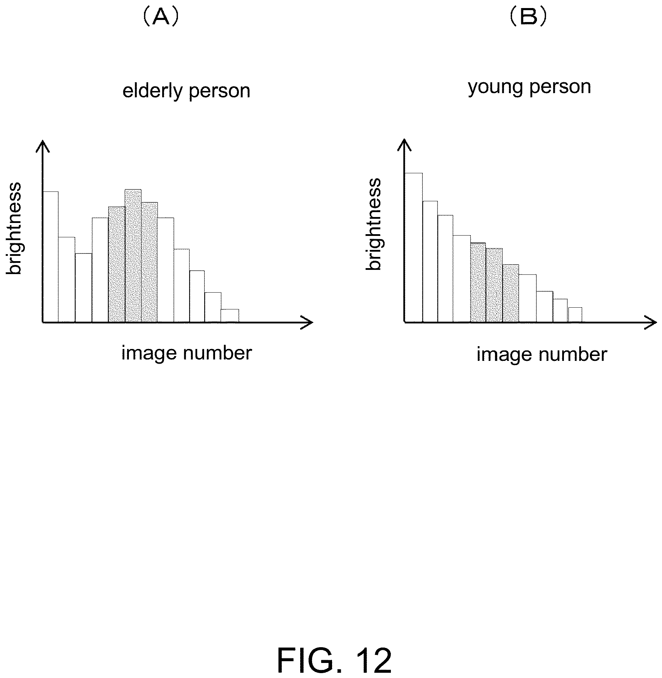

A histogram representing the brightness distribution can also be used to evaluate the muscle quality. For example, as shown in FIGs. 12 (A) and 12 (B), since a ratio of the number of pixels to a specific brightness value is different between the elderly and the young, the muscle quality can be evaluated based on the ratio.

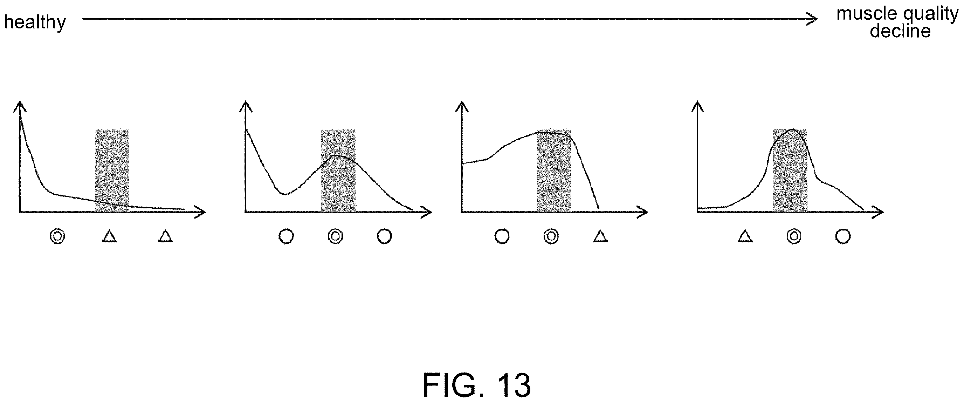

Alternatively, the muscle quality can be evaluated by a size relationship of the region of the brightness distribution. Since the brightness distribution differs as the muscle quality decreases from the healthy state, for example, as shown in FIG. 13, the brightness values in the gray area and the areas before and after the gray area are judged in 3 stages of ⊚ (abound), ∘ (intermediate), and △ (little), and the muscle quality is evaluated by the combination of the size relation. In each graph of FIG. 13, the vertical axis and the horizontal axis represent brightness and image number, respectively.

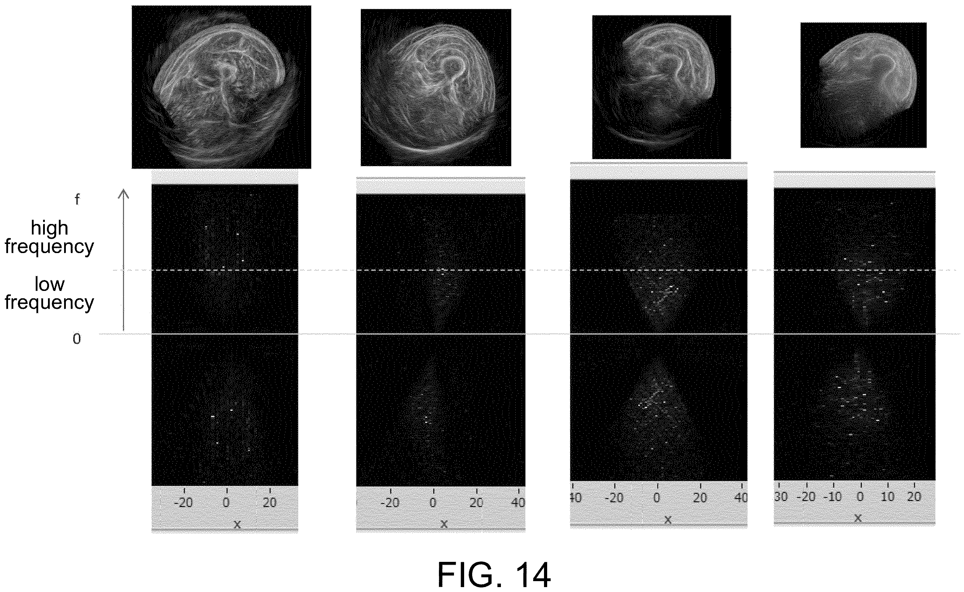

Alternatively, patterns and spatial frequencies can be used to evaluate muscle quality. For example, a two-dimensional fast Fourier transform (FFT: Fast Fourier Transform) is performed on a linear scan image. The upper four images shown in FIG. 14 are images before conversion, and the lower four images are images after conversion. In the normal state, a high frequency component of the brightness in the converted image is larger than a low frequency component, and the low frequency component is at a low level, but the distribution of the brightness moves to the low frequency side with the lowering of the muscle quality. Therefore, the muscle quality can be evaluated based on the high frequency component and the low frequency component. It should be noted that this evaluation method can improve the evaluation accuracy by additionally performing the above-mentioned muscle quality evaluation using the average value or histogram of brightness.

The cross-sectional area calculator 326 shown in FIG. 4 calculates the cross-sectional areas of the rectus femoris, vastus lateralis, vastus intermedius, and vastus medialis of the quadriceps femoris 92. The calculation result is an input to the muscle quality evaluation module 325 together with the evaluation result of the decision module 327. The decision module 327 also receives subject information T such as the age, sex, height, and weight of the body 9 stored in the recording module 34. The cross-sectional area calculator 326 and the subject information T may have any configuration.

The decision module 327 presents the evaluation result of the muscle quality, the muscle cross-sectional area, and reference data based on the subject information T inputted from the muscle quality evaluation module 325 and the cross-sectional area calculator 326, respectively. Further, the decision module 327 performs calculation (e.g., T-SCORE) in comparison with the reference data for determination. These results are output to the output module 33 and can be confirmed by the user.

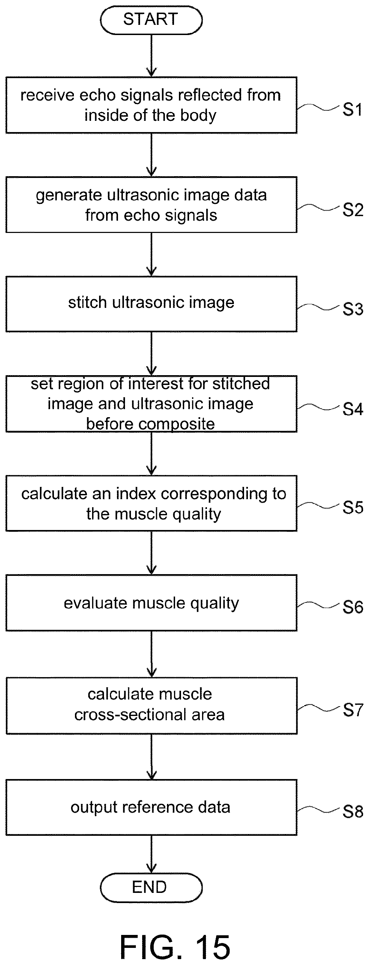

[ultrasonic analysis method] FIG. 15 is a flow chart showing a flow of processing in an ultrasonic analysis method according to the present embodiment. The ultrasonic analysis method according to the present embodiment can be implemented by the transmitter/receiver 2 and the ultrasonic analysis apparatus 3.

In step S1, the transmitter/receiver 2 shown in FIG. 1 transmits ultrasonic waves from different positions from the transducer of the ultrasonic probe 21 toward the muscles inside of the body 9, and receives the echo signals reflected from inside of the body 9 by the transducer of the ultrasonic probe 21. In this embodiment, the muscle is the quadriceps femoris 92. The transmitter/receiver 2 digitally converts the received echo signal and transmits it to the ultrasonic analysis apparatus 3, and the I/F module 35 of the ultrasonic analysis apparatus 3 receives the echo signal from the transmitter/receiver 2.

In step S2, the ultrasonic image generating module 321 shown in FIG. 4 generates an ultrasonic image of the inside of the body 9 based on the received echo signal. In the present embodiment, the ultrasonic image generating module 321 generates a linear scan image and a sector scan image as ultrasonic images.

In step S3, the image compositing module 322 stitches the ultrasonic images to generate a stitched image.

In step S4, the area setting module 323 sets a region of interest for the stitched image and the ultrasonic image before the stitching. In this embodiment, a region of interest is set for each of the rectus femoris, vastus lateralis, vastus intermedius, and vastus medialis constituting the quadriceps femoris 92.

In step S5, the index calculation module 324 calculates an index corresponding to the muscle quality based on the image corresponding to the secondary region of interest set for the ultrasonic image before the stitching. The index is, for example, an average value of brightness of an image.

In step S6, the muscle quality evaluation module 325 evaluates the muscle quality based on the index. The evaluation of the muscle quality can be performed based on the average value of the brightness, the histogram of the brightness, the ratio of the number of pixels of a specific brightness value, etc.

In step S7, the cross-sectional area calculating module 326 calculates a muscle cross-sectional area. In the present embodiment, the cross-sectional area calculation module 326 calculates the cross-sectional areas of the rectus femoris, vastus lateralis, vastus intermedius, and vastus medialis of the quadriceps femoris 92.

In step S7, the decision module 327 presents the evaluation result of the muscle quality, the muscle cross-sectional area, and reference data based on the subject information T.

[summary of the embodiments] According to the ultrasonic analysis apparatus 3 and the ultrasonic analysis method according to the present embodiment, it is possible to evaluate the muscle quality by analyzing an ultrasonic image without using a CT apparatus or an MRI apparatus having a complicated configuration as in the prior art. Therefore, it is possible to evaluate the muscle quality with a relatively simple constitution without accompanying exposure to possibly harmful radiations.

While the embodiments of the present invention have been described above, the present invention is not limited to the embodiments described above, and various modifications can be made provided they fall under the scope of the appended claims.

In the above embodiment, the index calculation module 324 calculates the index corresponding to the muscle quality from the secondary region of interest set for the ultrasonic image before synthesis, in accordance with the invention but the index could have been calculated from the region of interest set for the stitched image in an example outside the scope of the present invention. However, in this example, the brightness decreases according to the depth in the ultrasonic image, and thus, the brightness is affected in the stitched image. Therefore, in accordance with the invention, the index is calculated from the secondary region of interest set for the ultrasonic image before synthesis.

Further, in the above embodiment, while one ultrasonic probe 21 is moved along the surface of the thigh 91, a plurality of ultrasonic images are acquired by transmitting ultrasonic waves from different positions toward the quadriceps femoris 92 a plurality of times, but the method for acquiring the plurality of ultrasonic images is not limited thereto. For example, a plurality of ultrasonic probes, similar to the ultrasonic probe 21, may be disposed around the thigh 91, and ultrasonic waves may be simultaneously transmitted from each ultrasonic probe toward the quadriceps femoris 92 to obtain the plurality of ultrasonic images.

Although the ultrasonic image generating module 321 generates the linear scan image and the sector scan image as ultrasonic images in the aforementioned embodiment, the ultrasonic image generating module 321 may generate only one of the linear scan image and the sector scan image in various other embodiments. Alternatively, the ultrasonic image generating module 321 may generate a convex image as an ultrasonic image. In this case, the region of interest may be set in the convex image, and the index corresponding to the muscle quality may be calculated from the image corresponding to the region of interest.

In the above embodiment, 200 linear scan images and 200 sector scan images are used for calculating the index respectively, but the number of ultrasonic images used for calculating the index is not particularly limited, and a part of the acquired ultrasonic images may be selected and used for calculating the index. For example, 50 images may be selected at predetermined intervals from the total 400 ultrasonic images arranged in an order of acquisition.

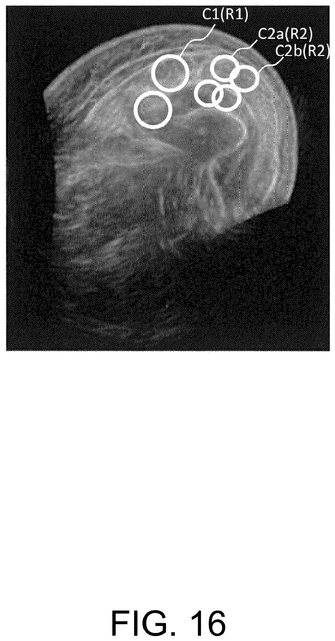

Further, when setting the region of interest in the stitched image, in the above embodiment, the quadriceps femoris 92 is divided into four regions of the rectus femoris, vastus lateralis, vastus intermedius, and vastus medialis, but it may be difficult to manually find the boundary of the region depending on the subject. Therefore, for example, as shown in FIG. 16, a typical position of the muscle may be surrounded by a frame having a simple shape such as a circular frame, and an area within the frame may be set as a region of interest. In the example shown in FIG. 16, a circular frame C1 including the rectus femoris is set as a region of interest R1. In the case of a muscle having an elongated cross-sectional shape such as the vastus lateralis muscle, a region within the muscle may be surrounded by a plurality of circular frames C2a and C2b, and a region within the circular frames C2a and C2b may be set as a region of interest R2.

When the quadriceps femoris 92 is used as the analysis subject of the muscle quality, one region of interest may be set for the entire quadriceps femoris 92, or at least one of the rectus femoris, vastus lateralis, vastus intermedius, and vastus medialis constituting the quadriceps femoris 92.

In the above embodiment, the analysis subject of the muscle is the quadriceps femoris 92, but the present invention is not limited thereto, and any muscle such as the back of the thigh 91, the calf, and the upper arm muscle can be included in the analysis subject.

Further, in the above embodiment, in order to obtain an ultrasonic image corresponding to a plurality of mutually different positions on the surface of the body 9, the ultrasonic wave is intermittently transmitted from the ultrasonic probe 21 while the ultrasonic probe 21 is moved along the surface of the body 9, the embodiments for obtaining ultrasound images are not limited thereto. For example, a plurality of ultrasonic probes 21 may be arranged in the body 9, and ultrasonic waves may be transmitted simultaneously from each ultrasonic probe 21.

### DESCRIPTION OF REFERENCE CHARACTERS

- 1: ultrasonic analysis system
- 2: transmitter/receiver
- 21: ultrasonic probe
- 22: transmitting and receiving module
- 23: A/D conversion module
- 24: probe adapter
- 3: ultrasonic analysis apparatus
- 31: input module
- 32: signal processing module
- 321: ultrasonic image generating module
- 322: image compositing module
- 323: area setting module
- 324: index calculation module
- 325: muscle quality evaluation module
- 326: cross-sectional area calculator
- 327: decision module
- 33: output module
- 34: recording module
- 35: interface module
- 9: body
- 91: thigh
- 92: quadriceps femoris
- 93: zonal region
- 93a: zonal region
- 93b: zonal region
- 93c: zonal region
- 94: fan-shaped region
- C1: circular frame
- C2a: circular frame
- C2b: circular frame
- Li1: linear scan image
- Li2: linear scan image
- Li3: linear scan image
- P: ultrasonic analysis program
- R1 - R4: regions of interest
- R 11 to R 14: regions of interest
- R 21 to R 24: regions of interest
- Se: sector scan image
- T: subject information

## Claims

1. An ultrasonic analysis apparatus (3), comprising:
an interface module (35) configured to receive signals from a transmitter/receiver (2) which receives ultrasonic waves transmitted from a plurality of mutually different positions on a surface of a body (9) toward a muscle inside the body (9) and reflected from inside the body (9);
an ultrasonic image generating module (321) configured to generate a plurality of ultrasonic images corresponding to the respective positions based on the signals inputted from the interface module (35);
an image compositing module (322) configured to stitch the plurality of ultrasonic images at the respective positions to generate a stitched image;
an area setting module (323) configured to both set a region of interest for the stitched image and a secondary region of interest for at least one ultrasonic image of the plurality of ultrasonic images before the stitch, the region of interest set for the stitched image and each secondary region of interest set for the at least one ultrasonic image before the stitch corresponding to each other; and
an index calculation module (324) configured to calculate an index related to the muscle, the index corresponding to a muscle quality of the muscle, from the secondary region(s) of interest set for the at least one ultrasonic image before the stitch.

2. The ultrasonic analysis apparatus (3) of claim 1, wherein:
the plurality of ultrasonic images include at least one of a linear scan image and a sector scan image.

3. The ultrasonic analysis apparatus (3) any one of claims 1 to 2, wherein:
the index is an average value of a brightness of the secondary region(s) of interest.

4. The ultrasonic analysis apparatus (3) any one of claims 1 to 3, wherein:
the muscle is a quadriceps femoris (92).

5. The ultrasonic analysis apparatus (3) of claim 4, wherein:
the area setting module (323) sets the region of interest and secondary region(s) of interest for each of a rectus femoris, a vastus lateralis, a vastus intermedius, and a vastus medialis of the quadriceps femoris (92), and
the index calculation module (324) calculates the index related to each muscle of the rectus femoris, the vastus lateralis, the vastus intermedius, and the vastus medialis.

6. The ultrasonic analysis apparatus (3) of claim 5, wherein:
the plurality of ultrasonic images include a sector scan image and a linear scan image, and
the index calculation module (324) calculates the index related to each muscle of the vastus lateralis and the vastus intermedius from the secondary region(s) of interest set in the linear scan image, and calculates the index related to each muscle of the rectus femoris and the vastus medialis from the secondary region(s) of interest set in the sector scan image.

7. The ultrasonic analysis apparatus (3) any one of claims 1 to 6, wherein:
the area setting module (323) extracts a mask image comprising the region of interest set in the stitched image from the stitched image and sets the secondary region of interest for the at least one ultrasonic image before the stitch by overlapping the mask image on the ultrasonic image

8. An ultrasonic analysis method, comprising the steps of:
receiving signals from a transmitter/receiver (2) which receives ultrasonic waves transmitted from a plurality of mutually different positions on a surface of a body (9) toward a muscle inside the body (9) and reflected from inside the body (9);
generating a plurality of ultrasonic images corresponding to the respective positions based on the received signals;
stitching the plurality of ultrasonic images at the respective positions to generate a stitched image;
setting both a region of interest for the stitched image and a secondary region of interest for at least one ultrasonic image of the plurality of ultrasonic images before the stitch, the region of interest set for the stitched image and each secondary region of interest set for the at least one ultrasonic image before the stitch corresponding to each other; and
calculating an index related to the muscle, the index corresponding to a muscle quality of the muscle, from the secondary region(s) of interest set for the at least one ultrasonic image before the stitch.

9. An ultrasonic analysis program configured to cause a computer to execute processing, the computer controlling an ultrasonic analysis apparatus according to any one of claims 1 to 7, which program comprises instructions, which when run on the computer, cause the ultrasonic analysis apparatus to carry out the method of claim 8.

## Patentansprüche

1. Ultraschallanalysevorrichtung (3), aufweisend:
ein Schnittstellenmodul (35), das so konfiguriert ist, dass es Signale von einem Sender/Empfänger (2) empfängt, der von einer Vielzahl von voneinander verschiedenen Positionen auf einer Oberfläche eines Körpers (9) in Richtung eines Muskels im Innern des Körpers (9) gesendete und aus dem Inneren des Körpers (9) reflektierte Ultraschallwellen empfängt;
ein Ultraschallbild-Erzeugungsmittel (321), das so konfiguriert ist, dass es eine Vielzahl von Ultraschallbildern entsprechend den jeweiligen Positionen auf der Grundlage der vom Schnittstellenmodul (35) eingespeisten Signale erzeugt;
ein Bildzusammensatzungsmodul (322), das so konfiguriert ist, dass es die Vielzahl von Ultraschallbildern an den jeweiligen Positionen zusammenfügt, um ein zusammengefügtes Bild zu erzeugen;
ein Bereichseinstellungsmodul (323), das so konfiguriert ist, dass es sowohl einen Bereich von Interesse für das zusammengefügte Bild als auch einen sekundären Bereich von Interesse für zumindest ein Ultraschallbild der Vielzahl von Ultraschallbildern vor der Zusammenfügung einstellt, wobei der für das zusammengefügte Bild eingestellte Bereich von Interesse und jeder, für das zumindest eine Ultraschallbild vor der Zusammenfügung eingestellte sekundäre Bereich von Interesse einander entsprechen; und
ein Indexberechnungsmodul (324), das so konfiguriert ist, dass es einen Index in Bezug auf den Muskel, wobei der Index einer Muskelqualität des Muskels entspricht, aus dem (den) für das zumindest eine Ultraschallbild vor der Zusammenfügung eingestellten sekundären Bereich(en) von Interesse berechnet

2. Ultraschallanalysevorrichtung (3) nach Anspruch 1, wobei
die Vielzahl von Ultraschallbildern ein Bild eines linearen Scans und/oder ein Bild eines Sektor-Scans umfasst.

3. Ultraschallanalysevorrichtung (3) nach einem der Ansprüche 1 bis 2, wobei
der Index ein Durchschnittswert einer Helligkeit des (der) sekundären Bereichs (Bereiche) von Interesse ist.

4. Ultraschallanalysevorrichtung (3) nach einem der Ansprüche 1 bis 3, wobei
es sich bei dem Muskel um einen Quadrizeps femoris (92) handelt.

5. Ultraschallanalysevorrichtung (3) nach Anspruch 4, wobei
das Bereichseinstellungsmodul (323) den Bereich von Interesse und den (die) sekundären Bereich(e) von Interesse für jeden eines Rectus femoris, eines Vastus lateralis, eines Vastus intermedius und eines Vastus medialis des Quadrizeps femoris (92) einstellt und
das Indexberechnungsmoduls (324) den Index in Bezug auf jeden Muskel des Rectus femoris, des Vastus lateralis, des Vastus intermedius und des Vastus medialis berechnet.

6. Ultraschallanalysevorrichtung (3) nach Anspruch 5, wobei
die Vielzahl von Ultraschallbildern ein Bild eines Sektor-Scans und ein Bild eines linearen Scans umfasst und
das Indexberechnungsmodul (324) den Index in Bezug auf jeden Muskel des Vastus lateralis und des Vastus intermedius aus dem (den) im Bild eines linearen Scans eingestellten sekundären Bereich(en) von Interesse berechnet und den Index in Bezug auf jeden Muskel der Rectus femoris und des Vastus medialis auf dem (den) im Bild eines Sektor-Scans eingestellten sekundären Bereich(en) von Interesse berechnet.

7. Ultraschallanalysevorrichtung (3) nach einem der Ansprüche 1 bis 6, wobei
das Bereichseinstellungsmodul (323) ein Maskenbild mit dem Bereich von Interesse, der im zusammengefügten Bild eingestellt ist, aus dem zusammengefügten Bild extrahiert und den sekundären Bereich von Interesse für das zumindest eine Ultraschallbild vor der Zusammenfügung einstellt, indem das Maskenbild auf dem Ultraschallbild überlagert wird.

8. Ultraschallanalyseverfahren, aufweisend die Schritte:
Empfangen von Signalen von einem Sender/Empfänger (2), der von einer Vielzahl von voneinander verschiedenen Positionen auf einer Oberfläche eines Körpers (9) in Richtung eines Muskels im Innern des Körpers (9) gesendete und aus dem Inneren des Körpers (9) reflektierte Ultraschallwellen empfängt;
Erzeugen einer Vielzahl von Ultraschallbildern entsprechend den jeweiligen Positionen auf der Grundlage der empfangenen Signale;
Zusammenfügen der Vielzahl von Ultraschallbildern an den jeweiligen Positionen, um ein zusammengefügtes Bild zu erzeugen;
Einstellen sowohl eines Bereichs von Interesse für das zusammengefügte Bild als auch eines sekundären Bereichs von Interesse für zumindest ein Ultraschallbild der Vielzahl von Ultraschallbildern vor der Zusammenfügung, wobei der für das zusammengefügte Bild eingestellte Bereich von Interesse und jeder, für das zumindest eine Ultraschallbild vor der Zusammenfügung eingestellte sekundäre Bereich von Interesse einander entsprechen; und
Berechnen eines Index in Bezug auf den Muskel, wobei der Index einer Muskelqualität des Muskels entspricht, aus dem (den) für das zumindest eine Ultraschallbild vor der Zusammenfügung eingestellten sekundären Bereich(en) von Interesse.

9. Ultraschallanalyseprogramm, das so konfiguriert ist, dass es einen Computer veranlasst, eine Verarbeitung auszuführen, wobei der Computer eine Ultraschallanalysevorrichtung nach einem der Ansprüche 1 bis 7 steuert, welches Programm Anweisungen aufweist, die, wenn sie auf dem Computer ausgeführt werden, veranlassen, dass die Ultraschallanalysevorrichtung das Verfahren nach Anspruch 8 ausführt.

## Revendications

1. Appareil d'analyse ultrasonore (3), comprenant :
un module d'interface (35) configuré pour recevoir des signaux en provenance d'un émetteur/récepteur (2) qui reçoit des ondes ultrasonores émises à partir d'une pluralité de positions mutuellement différentes sur une surface d'un corps (9) vers un muscle à l'intérieur du corps (9) et réfléchies depuis l'intérieur du corps (9) ;
un module de génération d'images ultrasonores (321) configuré pour générer une pluralité d'images ultrasonores correspondant aux positions respectives sur la base des signaux entrés en provenance du module d'interface (35) ;
un module de composition d'images (322) configuré pour assembler la pluralité d'images ultrasonores aux positions respectives pour générer une image assemblée ;
un module de définition de zone (323) configuré pour définir à la fois une région d'intérêt pour l'image assemblée et une
région d'intérêt secondaire pour au moins une image ultrasonore de la pluralité d'images ultrasonores avant l'assemblage, la région d'intérêt définie pour l'image assemblée et chaque région d'intérêt secondaire définie pour l'au moins une image ultrasonore avant l'assemblage correspondant l'une à l'autre ; et
un module de calcul d'indice (324) configuré pour calculer un indice se rapportant au muscle, l'indice correspondant à une qualité musculaire du muscle, à partir de la ou des région(s) d'intérêt secondaire(s) définie(s) pour l'au moins une image ultrasonore avant l'assemblage.

2. Appareil d'analyse ultrasonore (3) selon la revendication 1, dans lequel :
la pluralité d'images ultrasonores comportent au moins l'une parmi une image de balayage linéaire et une image de balayage sectoriel.

3. Appareil d'analyse ultrasonore (3) selon l'une des revendications 1 à 2, dans lequel :
l'indice est une valeur moyenne d'une luminosité de la ou des région(s) d'intérêt secondaire(s).

4. Appareil d'analyse ultrasonore (3) selon l'une des revendications 1 à 3, dans lequel :
le muscle est un muscle quadriceps fémoral (92).

5. Appareil d'analyse ultrasonore (3) selon la revendication 4, dans lequel :
le module de définition de zone (323) définit la région d'intérêt et une ou des région(s) d'intérêt secondaire(s) pour chacun parmi un muscle droit fémoral, un muscle vaste latéral, un muscle vaste intermédiaire et un muscle vaste médial du muscle quadriceps fémoral (92), et
le module de calcul d'indice (324) calcule l'indice se rapportant à chaque muscle parmi le muscle droit fémoral, le muscle vaste latéral, le muscle vaste intermédiaire et le muscle vaste médial.

6. Appareil d'analyse ultrasonore (3) selon la revendication 5, dans lequel :
la pluralité d'images ultrasonores comportent une image de balayage sectoriel et une image de balayage linéaire, et
le module de calcul d'indice (324) calcule l'indice se rapportant à chaque muscle parmi le muscle vaste latéral et le muscle vaste intermédiaire à partir de la ou des région(s) d'intérêt secondaire(s) définie(s) dans l'image de balayage linéaire, et calcule l'indice se rapportant à chaque muscle parmi le muscle droit fémoral et le muscle vaste médial à partir de la ou des région(s) d'intérêt secondaire(s) définie(s) dans l'image de balayage sectoriel.

7. Appareil d'analyse ultrasonore (3) selon l'une des revendications 1 à 6, dans lequel :
le module de définition de zone (323) extrait une image de masque comprenant la région d'intérêt définie dans l'image assemblée à partir de l'image assemblée et définit la région d'intérêt secondaire pour l'au moins une image ultrasonore avant l'assemblage par la superposition de l'image de masque sur l'image ultrasonore.

8. Procédé d'analyse ultrasonore, comprenant les étapes de :
la réception de signaux en provenance d'un émetteur/récepteur (2) qui reçoit des ondes ultrasonores émises à partir d'une pluralité de positions mutuellement différentes sur une surface d'un corps (9) vers un muscle à l'intérieur du corps (9) et réfléchies depuis l'intérieur du corps (9) ;
la génération d'une pluralité d'images ultrasonores correspondant aux positions respectives sur la base des signaux reçus ;
l'assemblage de la pluralité d'images ultrasonores aux positions respectives pour générer une image assemblée ;
la définition à la fois d'une région d'intérêt pour l'image assemblée et d'une région d'intérêt secondaire pour au moins une image ultrasonore de la pluralité d'images ultrasonores avant l'assemblage, la région d'intérêt définie pour l'image assemblée et chaque région d'intérêt secondaire définie pour l'au moins une image ultrasonore avant l'assemblage correspondant l'une à l'autre ; et
le calcul d'un indice se rapportant au muscle, l'indice correspondant à une qualité musculaire du muscle, à partir de la ou des région(s) d'intérêt secondaire(s) définie(s) pour l'au moins une image ultrasonore avant l'assemblage.

9. Programme d'analyse ultrasonore configuré pour amener un ordinateur à exécuter un traitement, l'ordinateur commandant un appareil d'analyse ultrasonore selon l'une des revendications 1 à 7, lequel programme comprend des instructions, qui lors de leur lancement sur l'ordinateur, amènent l'appareil d'analyse ultrasonore à mettre en œuvre le procédé selon la revendication 8.
